# EUROPEAN PATENT SPECIFICATION

(11) **EP 2 358 268 B1**
(45) Date of publication and mention of the grant of the patent: **27.03.2019**
(21) Application number: 08878197.6
(22) Date of filing: 18.11.2008
(51) Int. Cl.: A61F 5/56, A61N 1/36, A61B 5/04, A61N 1/372, A61B 5/00, A61B 5/08, A61B 5/087

(54) **DEVICE FOR DETECTING SLEEP APNEA EVENTS BY MONITORING THE INTERNAL BRANCH OF THE SUPERIOR LARYNGEAL NERVE**
VORRICHTUNG ZUR ERFASSUNG VON SCHLAFAPNÖEREIGNISSEN DURCH ÜBERWACHUNG DES INTERNEN ZWEIGS DES OBEREN KEHLKOPFNERVS
DISPOSITIF DE DÉTECTION DES ÉVÉNEMENTS D'APNÉE DU SOMMEIL PAR SURVEILLANCE DE LA BRANCHE INTERNE DU NERF LARYNGÉ SUPÉRIEUR

(43) Date of publication of application: 24.08.2011
(73) Proprietor: MED-EL Elektromedizinische Geräte GmbH, 6020 Innsbruck (AT)
(72) Inventor: WILSON, Willard, Québec, Québec G1Y 3C6 (CA); KAMELI, Nader, Hugo, Minnesota 55038 (US)
(74) Representative: Lucke, Andreas
(86) International application number: PCT/CA2008/002036
(87) International publication number: WO 2010/057286

(56) References cited:
- WO-A1-2008/046190
- WO-A1-2008/046190
- WO-A2-00/06249
- WO-A2-2008/005903
- US-A1- 2007 150 006

## Description

### TECHNICAL FIELD

The present invention relates to a method and device for the detection, identification and treatment of sleep apnea/hypopnea.

### BACKGROUND

Sleep apnea/hypopnea affects around 5% of the adult U.S. population. Its short-term effects consist of complete (apnea) or partial (hypopnea) termination of airflow, decreased oxygen in the blood, increased CO₂ in the blood, interrupted sleep, and excessive daytime sleepiness. Long-term effects may include hypertension, diabetes, heart attack, stroke, arrhythmia and brain damage.

The principal forms of sleep apnea are: 1) obstructive sleep apnea (OSA), characterized by a physical blockage of the upper airway during sleep, 2) central sleep apnea (CSA), caused by a decreased central respiratory drive during sleep, and 3) mixed sleep apnea, which includes components of both OSA and CSA. OSA is the most common and dangerous of all sleep-related breathing disorders. While CSA is uncommon in its pure form, it is prevalent in patients with congestive heart failure, as a component of Cheyne-Stokes respiration.

The obstructive component in OSA is related to decreased tone in the upper airway as the muscles relax during sleep. During normal respiration, upper airway patency is maintained by the negative pressure reflex, which activates pharyngeal dilators in response to negative transthoracic pressure during inspiration. In apneic patients, the negative pressure reflex is insufficient to maintain patency during sleep. Here, the negative pressure created during inspiration is sufficient to constrict or collapse the lumen of the flaccid airway.

The treatment of choice for sleep apnea is continuous positive air pressure (CPAP). Basically, CPAP maintains an open airway by inflating it with pressurized air through a nose or face mask. Used properly, CPAP is 100% effective for treating OSA. Although CSA has a neurological origin, it has also been successfully treated with positive air pressure. Despite its efficacy, however, CPAP treatment is poorly tolerated by sleep apnea patients. In one recent survey, CPAP non-compliance (less than 4h/night) was reported in between 46% and 83% of patients [1]. Reasons for non-compliance include discomfort, claustrophobia, pressure sores, dry nose or mouth, and machine noise.

The most common alternative to CPAP is a surgical removal of the uvula, caudal soft palate, and tonsils. This procedure has a success rate of about 50%. Other surgical treatments, such as tongue reduction, advancement of the tongue, tracheostomy, or implants to stiffen the soft palate have limited benefit relative to their invasiveness, risk, and irreversibility. Non-surgical approaches such as weight loss, medication, changes in sleeping position or dental appliances also suffer from limited effectiveness or compliance.

Implantable medical devices are currently under investigation as a method to detect and/or treat sleep apnea. Such devices are similar in their general design to cardiac pacemakers and share in many of the advantages of this mature technology.

With regard to detection, implantable devices have been described that detect apnea by monitoring the bioelectric activity of the diaphragm, intercostal muscles, or their efferent nerves. Other devices monitor the bioelectric activity of upper airway muscles or their efferent nerves. Still others monitor implanted sensors for indications of, for example, thoracic pressure or blood oxygenation.

With regard to treatment, implantable devices have been described that terminate apnea using drug delivery, atrial overdrive pacing or electrical stimulation of the nerves or muscles that control respiratory activities. For OSA, electrical stimulation has been described that maintains patency by activating upper airway muscles or the efferent nerves controlling them. For CSA, treatments that elicit breathing by electrically stimulating the diaphragm, intercostal muscles, or their efferent nerves have been described.

WO 2008/046190 A1 is considered to represent the closest prior art, it discloses a method and apparatus for monitoring the respiratory activity of a subject comprising the steps of recording an electroneurogram signal from the vagus nerve of the subject, amplifying the electroneurogram signal, computing an amplitude envelope of the amplified electroneurogram signal, applying a matched filter to the amplitude envelope, and computing a time between successive peaks of the filtered amplitude envelope, wherein the time between the successive peaks is indicative of the respiratory activity of the subject.

WO 00/06249 A2 discloses an apparatus and method for treatment of obstructive sleep apnea by sensing hypoglossal nerve electroneurogram activity for purposes of detecting or predicting obstructive sleep apnea. The sensed hypoglossal nerve activity by itself is used to trigger functional electrical stimulation of the hypoglossal nerve in order to improve upper airway patency.

WO 2008/005903 A2 discloses an implantable apparatus for stimulating a hypoglossal nerve of a patient for the treatment of sleep disordered breathing. The system comprises an electrode positioned at least partially around the hypoglossal nerve and comprising a monitoring contract adapted to sense the electroneurogram activity of the hypoglossal nerve and at least one stimulating contact adapted to electrically stimulate the hypoglossal nerve. The system further comprises a controller operatively coupled to the monitoring contact and the stimulating contact. The monitoring contact monitors the electroneurogram activity of the hypoglossal nerve for a pre-inspiratory drive signal indicative of the breathing cycle and sends a signal to the controller. The controller operates to cause the stimulating electrodes to electrically stimulate the hypoglossal nerve in response to the pre-inspiratory drive signal to prevent occlusion of the patient's upper airway. This document further suggests observing intrathoracic pressure, but this pressure observation is not based on any electroneurogram signals, but based on separate pressure sensors implanted to the neck of the patient.

### SUMMARY

According to an illustrative example, there is provided a method for monitoring the respiratory activity of a subject, comprising the steps of:
recording an electroneurogram signal from the internal branch of the superior laryngeal nerve of the subject;
conditioning the electroneurogram signal;
computing an index of respiratory activity of the conditioned electroneurogram signal; and
reporting an occurrence of an apneic event when the index of respiratory activity meets at least one apnea criteria.

According to another illustrative example, the method further comprising the step of generating a stimulation signal which acts to increase airway patency or stimulate breathing following the reporting of the apneic event.

According to yet another illustrative example, the index of respiratory activity is computed by applying a rectification and bin-integrated algorithm to the conditioned electroneurogram signal.

According to a further illustrative example, the at least one apnea criteria includes a first criteria associated with obstructive sleep apnea and a second criteria associated with central sleep apnea, and wherein the reporting step includes reporting the apneic event as an obstructive sleep apnea event when the index of respiratory activity meets the first criteria and as a central sleep apnea event when the index of respiratory activity meets the second criteria.

According to a further still illustrative example, the method further comprises the step of reporting an occurrence of an obstructive sleep hypopnea event when the index of respiratory activity meets a third criteria associated with obstructive sleep hypopnea and reporting an occurrence of a central sleep hypopnea event when the index of respiratory activity meets a fourth criteria associated with central sleep hypopnea.

According to another illustrative example, there is provided a method for treating sleep apnea and/or hypopnea of a subject, comprising eliciting a reflexive pattern activity from the central nervous system of the subject following the detection of the sleep apnea event.

According to a further illustrative example, the reflexive pattern activity is swallowing and is elicited by stimulating the internal branch of the superior laryngeal nerve or the glossopharyngeal nerve with an electrical signal at a rate of about 20 Hz to 50 Hz.

According to the present invention, there is provided a system implementing the above described methods.

### BRIEF DESCRIPTION OF THE DRAWINGS

Non-limitative illustrative embodiments of the invention will now be described by way of example only with reference to the accompanying drawings, in which:
Figure 1 is a schematic representation of the human airway relevant to upper airway pressure as measured at the larynx during normal respiration;
Figure 2 is a graph of airway pressure measured at the larynx during the normal breathing process;
Figure 3 is a schematic representation of the human airway relevant to upper airway pressure as measured at the larynx during an obstructive sleep apnea (OSA) event;
Figure 4 is a graph of airway pressure measured at the larynx at the outset of an OSA event;
Figure 5 is a schematic representation of the human airway relevant to upper airway pressure as measured at the larynx during a central sleep apnea (CSA) event;
Figure 6 is a graph of airway pressure measured at the larynx at the outset of a CSA event;
Figure 7 is a graph of an index of respiratory activity (IRA) computed from the rectified and bin-integrated (RBI) electroneurogram of the iSLN during normal respiration;
Figure 8 is a graph of an index of respiratory activity (IRA) computed from an RBI electroneurogram of the ISLN at the outset of an OSA event;
Figure 9 is a graph of an alternative index of respiratory activity (IRA) computed from the duration of peaks in an RBI electroneurogram of the ISLN at the outset of an OSA event;
Figure 10 is a graph of a further alternative index of respiratory activity (IRA) computed from the interval between peaks in an RBI electroneurogram of the ISLN at the outset of an OSA event;
Figure 11 is a graph of an index of respiratory activity (IRA) computed from an RBI electroneurogram of the iSLN at the outset of a CSA event;
Figure 12 is a graph of an index of respiratory activity (IRA) computed from the rectified and bin-integrated (RBI) electroneurogram of the iSLN during normal respiration showing multiple apnea severity levels;
Figure 13 is a flow diagram depicting the detection, identification and treatment of sleep apnea process in accordance with a first illustrative embodiment of the present invention;
Figure 14 is a flow diagram depicting the detection, identification and treatment of sleep apnea and hypopnea process in accordance with a second illustrative embodiment of the present invention;
Figure 15 is a block diagram of an example of a neuromodulator for the detection, identification and treatment of sleep apnea.

### DETAILED DESCRIPTION

Generally stated, the non-limitative illustrative embodiment of the present invention provides a device for treating sleep dysfunctions such as sleep apnea/hypopnea by monitoring respiratory-related activity from nerve or muscle, interpreting these internal signals to detect and classify adverse events in the airway, and stimulating nerves or muscles to elicit appropriate corrective responses to adverse respiratory events.

In the detailed description, unless specified otherwise, reference to the term "apnea" is defined to mean either an obstructive, central, mixed, or complex episode of apnea or hypopnea, occurring during sleep or awake as in Cheynne-Strokes respiration.

### Normal respiration

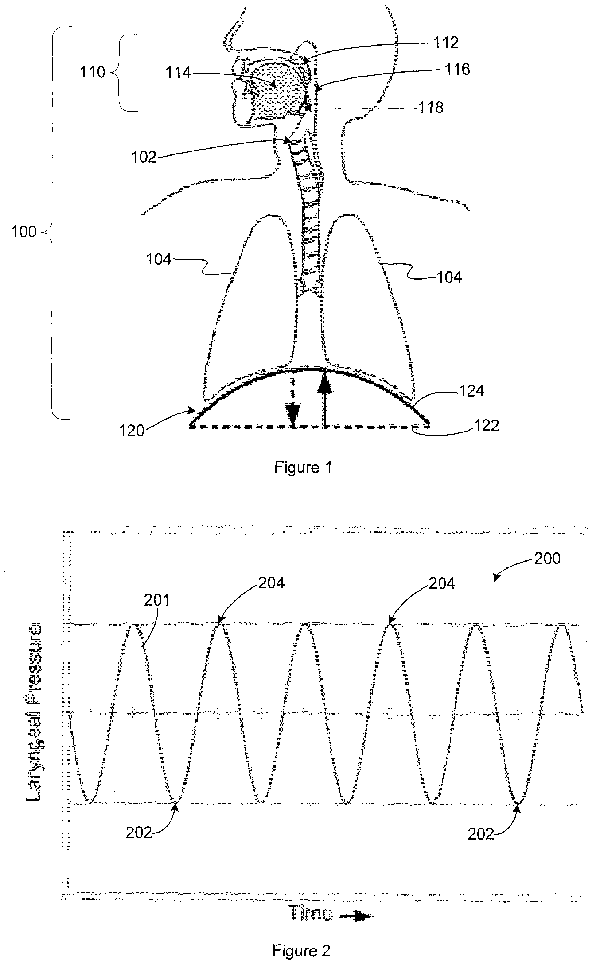

Referring to Figure 1, there is shown a schematic representation of the human airway 100 relevant to upper airway 110 pressure as measured at the larynx 102 during normal respiration. During normal inspiration, the diaphragm and intercostal muscles 120 contract 122, creating a negative pressure in the airway 100 and drawing air into the lungs 104. Expiration is typically passive, resulting from relaxation of the diaphragm and intercostal muscles 120 back to their resting position 124, and elastic recoil of the lungs 104. The amount of air flow produced by changing airway 100 pressure is influenced by resistance from the structures of the upper airway 110, including the soft palate 112, tongue 114, pharynx 116, and epiglottis 118.

Figure 2 shows a graph 200 of airway pressure 201 measured at the larynx 102 (see Figure 1) during the normal breathing process, comprising regular inspiration 202 and expiration 204 peaks of similar amplitude and frequency. Airway pressure at larynx 102 is transduced by mucosal mechanoreceptors that are sensitive to pressure and is communicated to the central nervous system via the internal branch of the superior laryngeal nerve (iSLN).

### Respiration during an OSA event

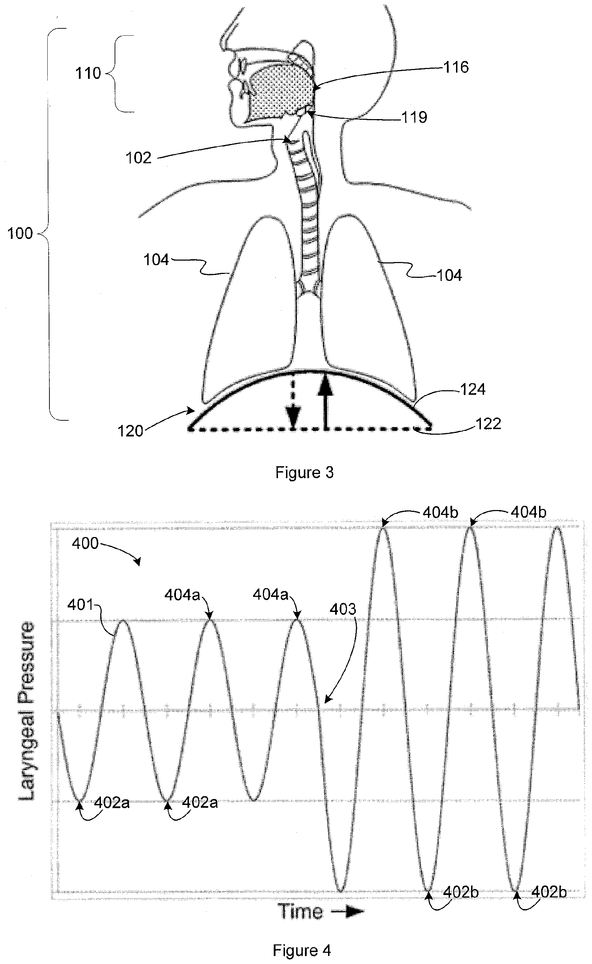

Referring to Figure 3 there is shown a schematic representation of the human airway 100 relevant to upper airway pressure as measured at the larynx during an OSA event. Here, a lack of muscle tone in the upper airway 110 allows pharyngeal structures 116 to partially or completely block the lumen 119 of the airway 100, particularly when subjects sleep on their back. Respiratory drive continues during the OSA event, the diaphragm and intercostal muscles 120 contract 122, creating a negative pressure in the airway 100 drawing flaccid pharyngeal structures 116 into the airway lumen 119.

Figure 4 shows a graph 400 of airway pressure 401 measured at the larynx 102 (see Figure 3) at the outset of an OSA event, comprising normal breathing process inspiration 402a and expiration 404a peaks before the OSA event and then inspiration 402b and expiration 404b peaks of a greater amplitude during the OSA event. This increase in the amplitude of the airway pressure 401 reflects continuing attempts on the part of the subject to breathe after airway obstruction, generating greater than normal airway pressures 401. The outset of the OSA event 403 can then be identified by the sudden increase in amplitude of the inspiration 402 and expiration 404 peaks of the airway pressure 401.

### Respiration during a CSA event

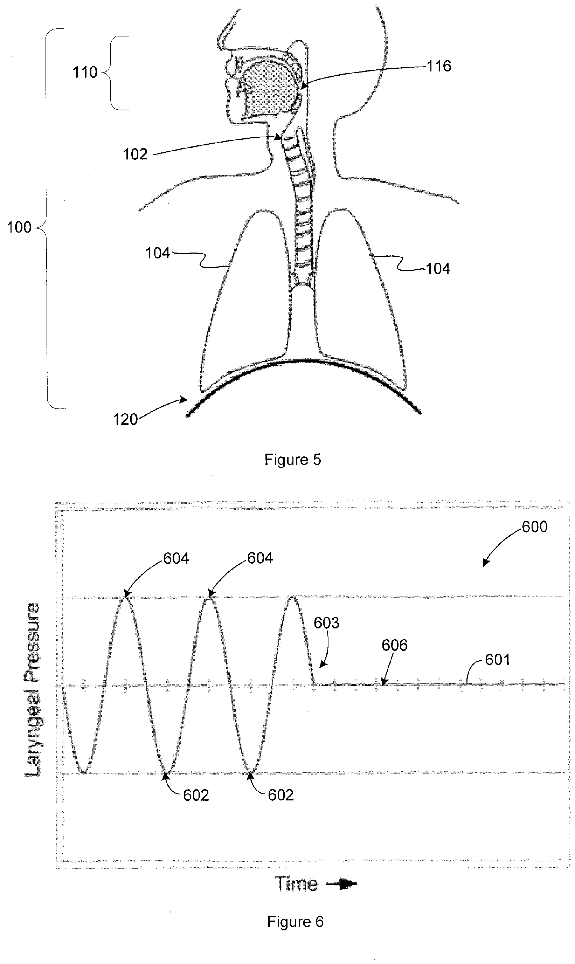

Referring to Figure 5, there is shown a schematic representation of the human airway 100 relevant to upper airway pressure as measured at the larynx during a CSA event. Here, the upper airway 110 remains open, but diminished central respiratory drive reduces or eliminates diaphragm 120 movement, and thus air flow during the CSA event.

Figure 6 shows a graph 600 of airway pressure 601 measured at the larynx 102 (see Figure 5) at the outset of a CSA event, comprising normal breathing process inspiration 602 and expiration 604 peaks before the CSA event and then an absence of, or very low amplitude, inspiration and expiration peaks 606 during the CSA event. Despite a patent upper airway 110, upper airway pressure 601 is not fully modulated after the onset of the CSA event and diminution of diaphragm movement. The outset of the CSA event 603 can then be identified by the sudden drop 606 in the amplitude of the inspiration 602 and expiration 604 peaks of the airway pressure 601.

### Detection and classification of apnea events

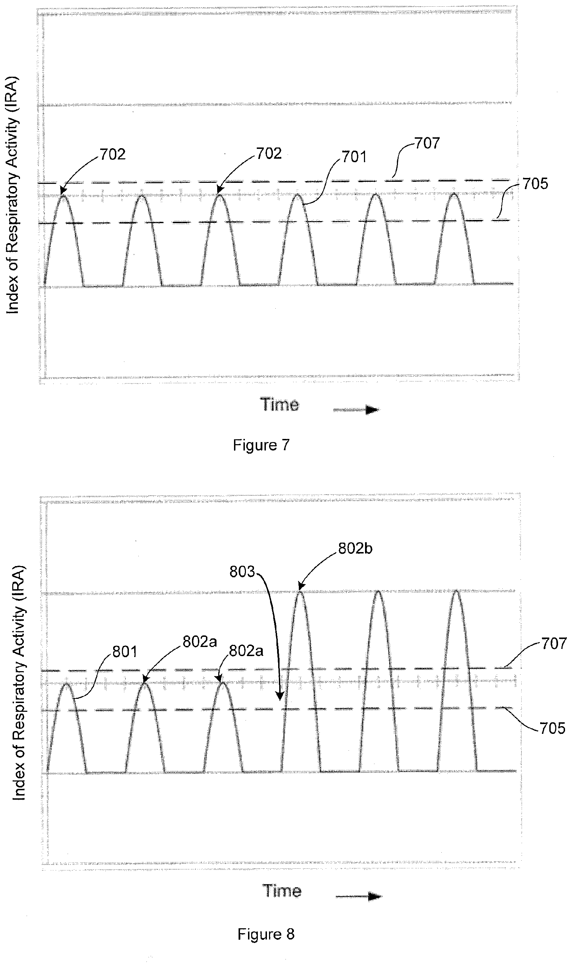

It has been discovered that the electroneurogram (ENG) of the internal branch of the superior laryngeal nerve (iSLN) is correlated with pressure in the upper airway 110 (see Figure 1). This relationship can be demonstrated by calculating an index of respiratory activity (IRA) that is indicative of the amplitude and timing of the ENG signal. For example, the IRA may be calculated by applying a rectification and bin-integration (RBI) algorithm to the amplified iSLN signal. Referring to Figure 7, during normal breathing, each peak 702 of the IRA 701 calculated using this method corresponds to a regular inspiration peak 202 of the airway pressure 201 measured at the larynx 102, as illustrated in Figure 2.

The amplitude of peaks in the IRA during each breath occurs within a normal range of amplitudes which may be determined using a calibration process during normal respiration of a given subject either empirically or using, for example, polysomnographic techniques. This range of amplitudes can be used to set upper 707 and lower 705 thresholds for apnea event detection. Peaks 702 outside of this normal range can be detected using simple fixed-level thresholds and defined as apneic events.

The upper 707 and lower 705 thresholds can further be used to classify, in real-time, a detected apneic event as being either an OSA event or a CSA event.

It is to be understood that although the above the IRA is calculated by applying a rectification and bin-integration (RBI) algorithm to the amplified iSLN signal, other signal processing algorithms may also be applied including: high pass filter, low pass filter, bandpass filter, notch filter, FIR filter, IIR filter, smoothing, moving average, Weiner (optimal) filter, rectification, bin-integration, multichannel noise reduction, principal components analysis, independent components analysis, wavelet analysis, Fourier transformation, matched filtering, variance/variance ratio calculations, or some combination of the above. The raw iSLN ENG waveform may also be used directly. IRAs based on neural network analyses, cluster analysis in multidimensional feature space, cluster cutting using k-means, Bayesian expectation-maximization, closest centers, or manual cluster cutting methods may also be used.

It is to be also understood that an IRA could be computed from any number of other iSLN ENG signal features that vary with respiratory state such as event or waveform timing, interval, amplitude, duration, rise time, fall time, slope, presence, absence, pattern, 1st derivative, 2nd derivative, 3rd derivative, root mean square amplitude, peak-to-peak amplitude, variance, statistical probability or probability relative to baseline or running average.

### OSA event

Referring to Figure 8, there is shown an example of peaks 802 of the IRA 801 at the outset of an OSA event, comprising normal breathing process inspiration related peaks 802a within the upper 707 and lower 705 thresholds before the OSA event and then inspiration related peaks 802b of an amplitude greater than the upper 707 threshold during the OSA event. The outset of the OSA event 803 can then be identified by the first crossing of the upper 707 threshold by the inspiration related peaks 802.

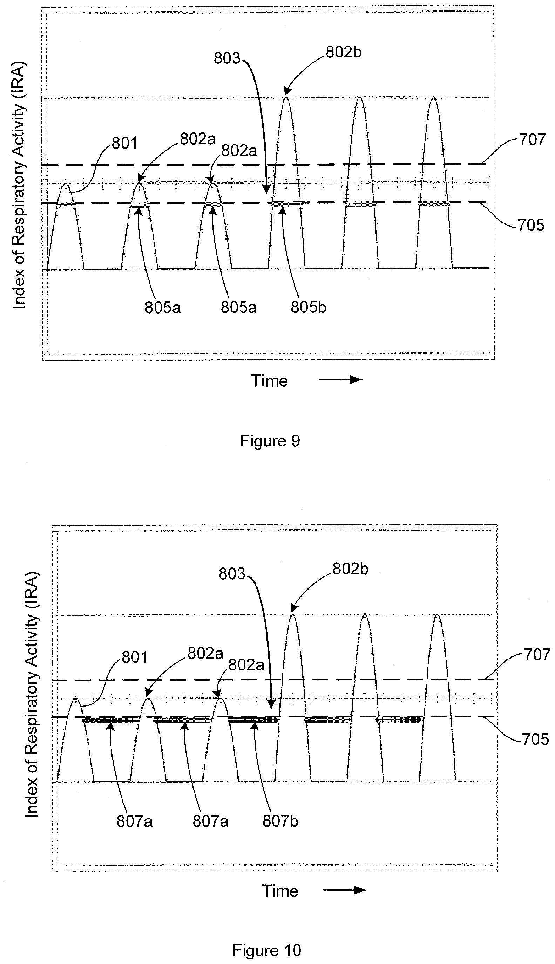

Alternatively, referring to Figure 9, it may be observed that at the outset of the OSA event 803 the durations 805b of the peaks 802b of the RBI ENG measured at a fixed level, for example threshold 705, are greater than the peak durations 805a of the peaks 802a measured during the normal breathing process. Accordingly, the peak durations of the RBI ENG may be used to calculate an IRA and identify the outset of an OSA event by setting an appropriate threshold.

Similarly, referring to Figure 10, it may be observed that at the outset of the OSA event 803 the intervals 807b between the peaks 802b of the RBI ENG measured at a fixed level, for example threshold 705, are smaller than the interpeak intervals 807a between the peaks 802a measured during the normal breathing process. Accordingly, the interpeak intervals of the RBI ENG may be used to calculate an IRA to identify the outset of an OSA event by setting an appropriate threshold.

### CSA event

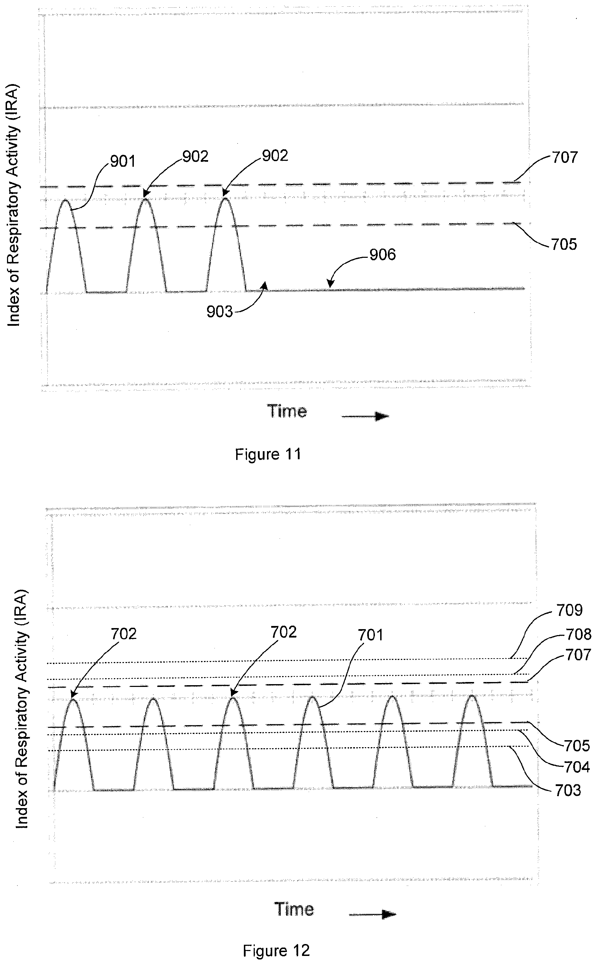

Referring to Figure 11, there is shown an example of peaks 902 of the IRA 901 at the outset of a CSA event, comprising normal breathing process inspiration related peaks 902 within the upper 707 and lower 705 thresholds before the CSA event and then inspiration related peaks 906 of an amplitude lower than the lower 705 threshold (or absence of peaks) during the CSA event. The outset of the CSA event 903 can then be identified by the first absence of crossing of the lower 705 threshold by the inspiration related peaks 902 for a set time period. This period of time may be set to represent the average time between one or more respiration cycle.

As is the case with OSA events (see Figures 9 and 10), other IRAs may be calculated in order to identify CSA events, such as the peak durations and interpeak intervals of the RBI ENG by setting appropriate levels and thresholds. It is to be understood that the absence of measurements at a specified level will indicate a CSA event.

It is also to be understood that detection of respiratory events in the IRA using methods other than fixed-level thresholding may be used, for example noise-tracking or other adaptive thresholds, energy or non-linear energy thresholds, or any variety of other detection operations on the raw or processed data.

### Apnea event severity

In an alternative embodiment, the severity of the apnea event may be determined by comparing the amplitude of the apneic IRA 801, 901 to that observed during normal breathing 701. More severe apnea is characterized by IRA peaks 802, 902 having amplitudes far from the upper 707 and lower 705 thresholds, while less severe apnea or hypopnea is characterized by IRA peaks 802, 902 having amplitudes just above or below the upper 707 and lower 705 thresholds. The level of apnea thus determined can be used to adjust the parameters and characteristics of the applied neurostimulation treatment. This may include changing the stimulation waveform, increasing or decreasing the stimulus amplitude, increasing or decreasing the number of stimuli delivered, selecting electrodes in specific locations or changing the number of stimulation electrodes used.

For example, referring to Figure 12, various OSA 707, 709 and CSA 705, 703 severity levels may be assigned corresponding thresholds. It is to be understood that the number of OSA and CSA severity levels may vary depending on the precision of the circuitry and/or algorithm used.

### Hypopnea

In a further alternative embodiment, apneic events may be further identified as OSA or obstructive sleep hypopnea (OSH) as well as CSA or central sleep hypopnea (CSH). For example, referring still to Figure 12, threshold 708 may be associated with OSH while threshold 707 may be associated with OSA, meaning that IRA peaks between thresholds 708 and 707 are identified as OSH while IRA peaks above threshold 707 are identified as OSA. Conversely, threshold 704 may be associated with CSH while threshold 705 may be associated with CSA, meaning that IRA peaks between thresholds 704 and 705 are identified as CSH while IRA peaks below threshold 705 are identified as CSA. The range of values for which IRA peaks are defined as OSH as opposed to OSA, as well as CSH as opposed to CSA, may be determined using a calibration process during abnormal respiration of a given subject using, for example, polysomnographic techniques.

It is to be understood that OSH, OSA, CSH and CSA may be subdivided into multiple severity levels depending on the precision of the circuitry and/or algorithm used.

As described above for the OSA and CSA event detection, the variation in IRAs calculated using algorithms other than RBI ENG may also be used to determine the severity of the apneic event.

Although peaks in the IRA coincident with negative pressure receptor activity are described above, it is to be understood that receptors sensitive to other stimuli and modalities, respiratory events, phases or features, and with afferents carried by other nerves may also used. This is meant to include mechanoreceptors sensitive to positive airway pressure, stretch, position, shear or slip, vibration, texture, touch, touch and pressure, muscle stretch, muscle "drive", air flow, blood pressure or osmolarity, chemoreceptors sensitive to CO₂, O₂, or pH, thermoreceptors sensitive to temperature or airflow, nociceptors sensitive to polymodal pain, or some combination of the above.

### Apneic event detection, identification and treatment

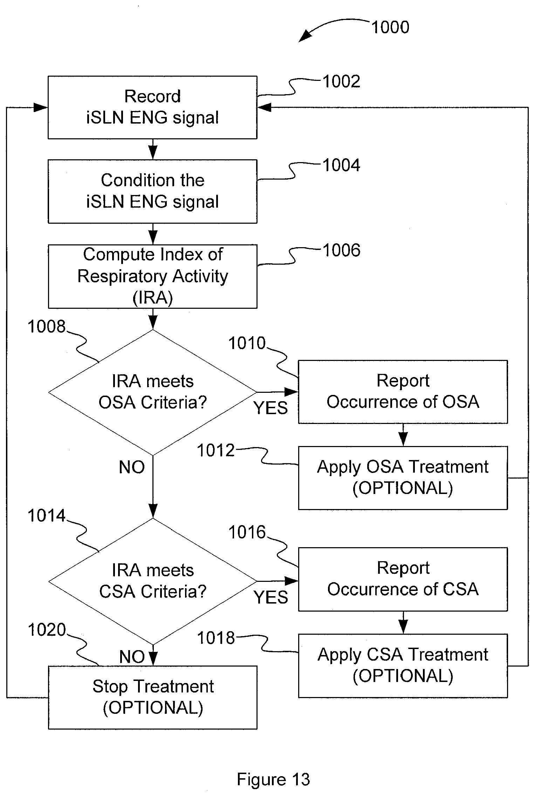

Referring to Figure 13, there is shown in a flow diagram a process 1000 for the detection and classification of apnea events in accordance with a first illustrative embodiment of the present invention. The steps composing the process are indicated by blocks 1002 to 1020.

The process 1000 starts at block 1002, where the iSLN ENG signal is recorded, after which, at block 1004, the iSLN ENG signal is conditioned (for example amplified).

At block 1006, an index of respiratory activity (IRA) is computed. The IRA is a measure of the iSLN ENG signal which varies with the respiratory activity of the subject and may be used to detect sleep apnea events through comparison with thresholds associated with normal respiratory activity. For example, the IRA may be the amplitude envelope computed by applying an RBI algorithm to the filtered and amplified iSLN ENG signal. This algorithm first rectifies the iSLN ENG signal and then sums the result in bins, essentially applying a low pass filter to the rectified signal. Alternatively, the IRA may be the root-mean-square or peak-to-peak amplitude of the iSLN ENG signal, the duration of peaks in the RBI iSLN ENG signal measured at a fixed level or the interval between peaks of the RBI iSLN ENG signal measured at a fixed level. It should be noted that the interpeak intervals are complimentary to the peak durations and as such an IRA based on interpeak interval values should be expressed as the inverse of the measured values.

Optionally, a moving average filter may then be applied to the IRA, for example a moving average filter spanning one second of data, and the result optimized using, for example, the solution to the Wiener-Hopf equation. The moving average filter helps to reduce the influence of variability inherent to iSLN ENG signals and its total length may be selected so as to be near the smallest feature (peak width) to be detected.

At block 1008, the process 1000 verifies if the IRA meets the criteria defining OSA. For example, in the case where the IRA is the RBI ENG of the iSLN signal, OSA is defined as peak values exceeding the upper threshold 707 of the range of amplitudes observed during normal respiration as illustrated in Figure 7. It is to be understood that the criteria defining OSA will vary depending on the IRA used.

If the IRA meets the criteria of OSA, the process 1000 proceeds to block 1010 where an OSA event is reported. Then, optionally, at block 1012, airway opening stimulation may be triggered in response to the detection of the OSA event.

The airway opening stimulation may act to replace or augment the negative pressure reflex in response to the OSA event, by artificially improving airway patency during inspiration. The stimulation may take a number of different forms and is designed to remain below the arousal threshold of the sleeping subject. Possible targets for stimulation include specific nerves that control upper airway patency such as, for example, the hypoglossal or glossopharyngeal nerves, or their combination. Likewise, direct stimulation of specific muscles that control upper airway patency such as, for example, genioglossus, tensor palatini, or sternohyoid muscles, or their combination, can also be used. Treatment can be accomplished by applying stimulation individually to some or all of the muscles involved in airway patency, or individually to the nerves efferent to these muscles, or some combination of the thereof. Stimulation of activities or muscles or nerves that increases upper airway patency can be delivered phasically, in synchrony with inspiration or tonically, throughout the entire respiratory cycle.

In an alternative embodiment, the stimulation may be aimed at eliciting reflexive and pre-programmed coordinated activity from swallow-related central pattern generators in the central nervous system. For example, the iSLN can be electrically stimulated at a rate of about 20 Hz to 50 Hz in order to provoke a swallowing reflex. Similar stimulus protocols give rise to one or more complete sequences of pharyngeal swallow. During pharyngeal swallow, upper airway pressure increases from negative values up to atmospheric pressure, the muscles of larynx and pharynx are activated, and the pharynx and larynx close and then open. The entire coordinated pattern of pharyngeal muscle activation ends with an open pharynx. Stimulation of this pattern is designed to open a collapsed upper airway and restore airway patency. Stimulation of other patterned activities may also be used to increase airway patency, such as cough, yawn, gag, etc., as well as some combination of stimulated patterned activities. Stimulation of the swallow sequence may also be accomplished using other nerves, for example the glossopharyngeal nerve.

The process 1000 then proceeds back to block 1002 where the recording of the iSLN ENG signal continues.

At block 1014, the process 1000 verifies if the IRA meets the criteria defining CSA. For example, in the case where the IRA is the RBI ENG of the iSLN signal, CSA may be defined as peak values remaining below the lower threshold 705 of the range of amplitudes observed during normal respiration as illustrated in Figure 7. It is to be understood that the definition of CSA will vary depending on the IRA used.

If the IRA meets the criteria of CSA, the process 1000 proceeds to block 1016 where a CSA event is reported. Then, optionally, at block 1018, breathing stimulation may be triggered in response to the detection of the CSA event.

The breathing stimulation acts to replace or augment respiratory drive in response to the CSA event. The stimulation may take a number of different forms and is designed to remain below the arousal threshold of the sleeping subject. Possible targets for stimulation include specific nerves that control respiratory muscles such as, for example, phrenic or intercostal nerves, or their combination. Likewise, direct stimulation of specific muscles that control respiration such as, for example, diaphragm or intercostal respiratory muscles, or their combination, can also used. Treatment can be accomplished by applying stimulation to some or all of the muscles involved in respiration, or to some or all the nerves efferent to these muscles, or some combination of the above. Stimulation of activities of muscles or nerves that increase respiration could be delivered in synchrony with remaining or previous inspiratory activity.

In an alternative embodiment, the stimulation may be aimed at eliciting reflexive and pre-programmed coordinated activity from swallow-related central pattern generators in the central nervous system as previously described at block 1012.

The process 1000 then proceeds back to block 1002 where the recording of the iSLN ENG signal continues.

Optionally, at block 1020, the IRA signal corresponding to normal respiration, the process 1000 terminates any ongoing treatment.

The process 1000 then proceeds back to block 1002 where the recording of the iSLN ENG signal continues.

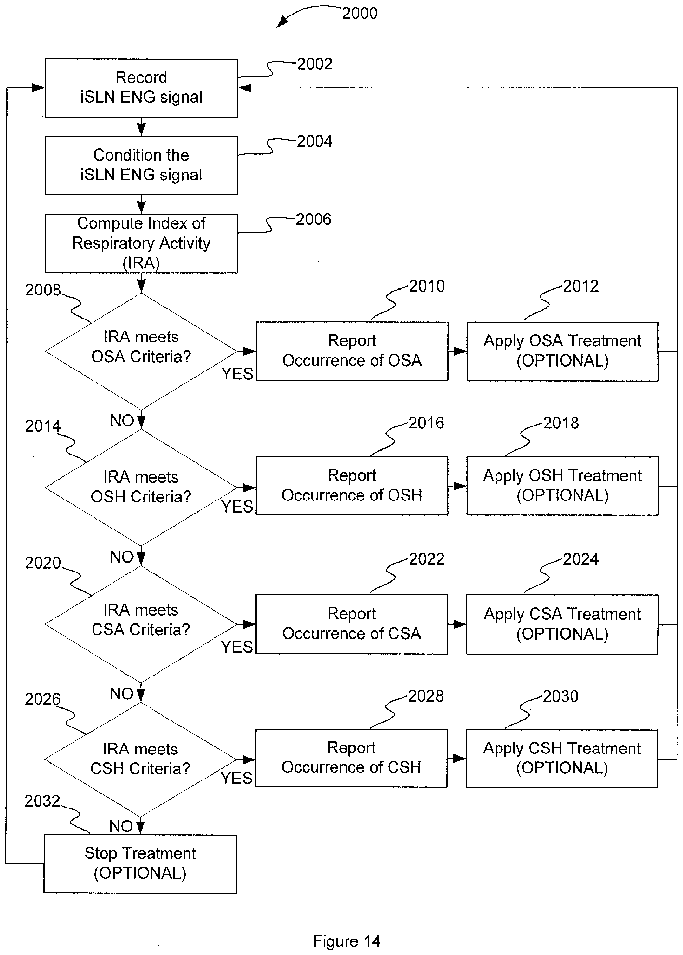

Referring to Figure 14, there is shown in a flow diagram a process 2000 for the detection and classification of apnea and hypopnea events in accordance with a second illustrative embodiment of the present invention. The steps composing the process are indicated by blocks 2002 to 2032.

The process 2000 starts at block 2002, where the iSLN ENG signal is recorded, after which, at block 2004, the iSLN ENG signal is conditioned (for example amplified).

At block 2006, an index of respiratory activity (IRA) is computed as previously described (see block 1006 of process 1000 from Figure 13).

At block 2008, the process 2000 verifies if the IRA meets the criteria defining OSA. For example, in the case where the IRA is the RBI ENG of the iSLN signal, the process 2000 verifies if peak values exceed threshold 708 associated with OSA (see Figure 12).

If the IRA meets the criteria defining OSA, the process 2000 proceeds to block 2010 where an OSA event is reported. Then, optionally, at block 2012, airway opening stimulation as previously described (see block 1012 of process 1000 from Figure 13) may be triggered in response to the detection of the OSA event.

The process 2000 then proceeds back to block 2002 where the recording of the iSLN ENG signal continues.

At block 2014, the process 2000 verifies if the IRA meets the criteria defining OSH. For example, in the case where the IRA is the RBI ENG of the iSLN signal, the process 2000 verifies if peak values are situated between thresholds 707 and 708 associated with OSH (see Figure 12).

If the IRA meets the criteria defining OSH, the process 2000 proceeds to block 2016 where an OSH event is reported. Then, optionally, at block 2018, airway opening stimulation as previously described (see block 1012 of process 1000 from Figure 13), but with adjusted parameters, may be triggered in response to the detection of the OSH event.

The process 2000 then proceeds back to block 2002 where the recording of the iSLN ENG signal continues.

At block 2020, the process 2000 verifies if the IRA meets the criteria defining CSA. For example, in the case where the IRA is the RBI ENG of the iSLN signal, the process 2000 verifies if peak values remain under the threshold 704 associated with CSA (see Figure 12).

If the IRA meets the criteria defining CSA, the process 2000 proceeds to block 2022 where a CSA event is reported. Then, optionally, at block 2024, breathing stimulation as previously described (see block 1018 of process 1000 from Figure 13) may be triggered in response to the detection of the CSA event.

The process 2000 then proceeds back to block 2002 where the recording of the iSLN ENG signal continues.

At block 2026, the process 2000 verifies if the IRA meets the criteria defining CSH. For example, in the case where the IRA is the RBI ENG of the iSLN signal, the process 2000 verifies if peak values are situated between thresholds 704 and 705 associated with CSH (see Figure 12).

If the IRA meets the criteria of CSH, the process 2000 proceeds to block 2028 where a CSH event is reported. Then, optionally, at block 2030, breathing stimulation as previously described (see block 1018 of process 1000 from Figure 13), but with adjusted parameters, may be triggered in response to the detection of the CSH event.

The process 2000 then proceeds back to block 2002 where the recording of the iSLN ENG signal continues.

It is to be understood that in other alternative embodiments, the detection and classification algorithm may further subdivide the OSH, OSA, CSH and CSA events into multiple severity levels, each level having associated stimulation parameter adjustments.

### Neuromodulator for the detection, identification and treatment of sleep apnea

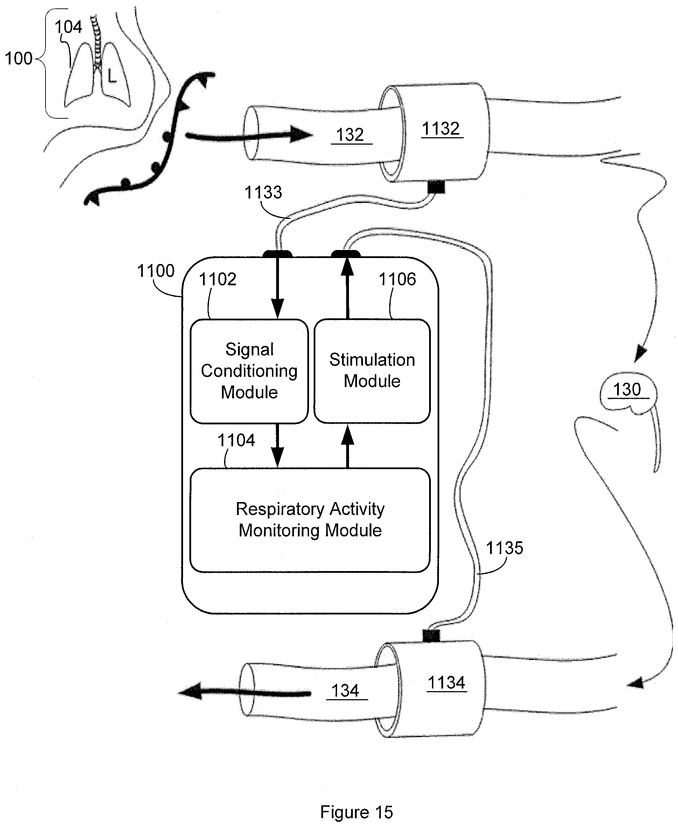

Referring to Figure 15, there is shown a block diagram of an example of a neuromodulator 1100 for detection, identification and treatment of sleep apnea by monitoring respiratory-related activity from nerve or muscle, interpreting these internal signals to detect and classify adverse events in the airway, and stimulating nerves or muscles to elicit appropriate corrective responses to adverse respiratory events.

In the illustrated example, apnea is detected and identified by monitoring respiratory-related activity from the internal branch of the superior laryngeal nerve (iSLN) 132. The iSLN carries afferents from receptors in the laryngeal mucosa toward the central nervous system 130. Other peripheral nerves carrying afferents modulated by respiratory condition may also be monitored, including the recurrent laryngeal nerve, the main branch of the SLN, the vagus nerve, the phrenic nerve, each nerve alone, or in combination with the other(s). Respiratory activity may also be monitored from nerves carrying efferent signals to muscles of the upper airway, diaphragm, or intercostal muscles, or by monitoring the activity of these respiratory muscles themselves, alone, or in some combination with other nerves or muscles modulated by respiratory activity.

The neuromodulator 1100 includes a signal conditioning module 1102, a respiratory activity monitoring module 1104 and a stimulation module 1106.

A recording electrode 1132 is placed in, around, or near a peripheral nerve that carries afferent neural activity from receptors in the upper airway 110 (see Figure 1) toward the central nervous system 130. One particular nerve that may be used is the iSLN 132. A lead 1133 connects the electrode 1132 to the signal conditioning module 1102.

It is to be understood that depending on the application there maybe multiple recording electrodes 1132 to simultaneously or sequentially monitor multiple signal sources. The recording electrode 1132 may also target other peripheral receptors that exhibit modulations of bioelectric potential correlated with respiration. Other receptors that may be monitored to determine respiratory condition include: mechanoreceptors sensitive to positive airway pressure, stretch, position, shear or slip, vibration, texture, touch, touch and pressure, muscle stretch, muscle "drive", air flow, blood pressure or osmolarity, chemoreceptors sensitive to CO₂, O₂, or pH, thermoreceptors sensitive to temperature or airflow, nociceptors sensitive to polymodal pain, or some combination of the above.

A stimulation electrode 1134 is placed in, around, or near, a target nerve or muscle depending on the type of stimulation used. A lead 1135 connects the stimulation electrode 1134 to the stimulation module 1106. The stimulation electrode 1134 may contain additional features allowing for enhanced current carrying capacity, selective stimulation using current steering, directionally selective stimulation of efferent or afferent fibers, or selectivity for stimulating axons of a particular diameter.

It is to be understood that there may be multiple targets for stimulation and that their selection may vary depending on the identified apneic event and the type of stimulation used. Furthermore, the stimulation may target the central nervous system 130 when the stimulation is aimed at eliciting reflexive and pre-programmed coordinated activity such as swallowing. It is further to be understood that in some alternative embodiment, a single electrode may be used both for the recording 1132 and the stimulation 1134 electrodes, for example when the iSLN is used for both recording and stimulation. Furthermore, multiple electrodes may be use, some or all of them being used both as recording 1132 and stimulation 1134 electrodes while others only as recording 1132 or stimulation 1134 electrodes.

In an alternative embodiment, iSLN ENG signals may be passed from electrode 1132 to the signal conditioning module 1102 wirelessly. Similarly, the stimulation signals from the stimulation module 1106 may be passed to the electrode 1134 wirelessly.

The electrodes 1132 and 1134 may be, for example, cuff electrodes. An example of a cuff electrode that may be used as electrodes 1132 and 1134 is disclosed in U.S. Patent No. 5,824,027 entitled "NERVE CUFF HAVING ONE OR MORE ISOLATED CHAMBERS", issued October 20, 1998, to Hoffer et al. It is to be understood that other types of electrodes, leads, probes, cuff-electrodes, etc., may be used as well. Other examples of cuff electrodes that may be used are disclosed in U.S. Patent Application Publication No. 2008/0065184 entitled "NERVE CUFF, METHOD AND APPARATUS FOR MANUFACTURING SAME", published March 13, 2008, by Hoffer et al. and PCT Patent Application Publication No. WO 2008/025155 entitled "NERVE CUFF INJECTION MOLD AND METHOD OF MAKING A NERVE CUFF", filed August 29, 2007, by Imbeau et al.

The signal conditioning module 1102 conditions the iSLN ENG signal, for example amplifying it, recorded by the first electrode 1132 and provides the conditioned iSLN ENG signal to the respiratory activity monitoring module 1104, which includes an algorithm that uses the conditioned iSLN ENG signal to monitor respiratory activity, detect apnea events before they result in arousal from sleep and identify the type of apnea event.

The signal conditioning module 1102 may include, without limiting the illustrative embodiment to these components, a signal amplifier and a rectifier circuit. Examples of amplifiers and rectifier circuit that may be used are respectively disclosed in U.S. Patent Application Publication No. 2006/0189881 entitled "IMPLANTABLE SIGNAL AMPLIFYING CIRCUIT FOR ELECTRONEUROGRAPHIC RECORDING", published August 24, 2006, by Baru Fassio and U.S. Patent No. 7,282,980 entitled "PRECISION RECTIFIER CIRCUIT FOR HIGH-DENSITY, LOW-POWER IMPLANTABLE MEDICAL DEVICE", issued October 16, 2007, to Baru Fassio.

The algorithm executed by the respiratory activity monitoring module 1104 implements blocks 1006 to 1010, 1014 and 1016 of process 1000 shown in Figure 13 or blocks 2006 to 2011 and 2014 to 2017 of process 2000 shown in Figure 14. Upon the detection of an apnea event, the respiratory activity monitoring module 1104 sends a trigger to the stimulation module 1106 along with an identification of the type of apnea event, i.e. OSH, OSA, CSH or CSA depending on the implemented algorithm, which generates a stimulation appropriate for the type of apnea event. Optionally, the respiratory activity monitoring module 1104 may also send an indication of the severity level of the apnea event, as well as timing information of previous or continuing respiration patterns, to the stimulation module 1106.

The respiratory activity monitoring module 1104 may optionally provide information about the respiratory activity of the subject, report sleep apnea events and/or allow remote modification of various criteria/thresholds through a communication link such as, for example, a radio frequency (RF) or infrared (IR) link (not shown).

The stimulation module 1106 implements the various stimulation strategies disclosed in blocks 1012 and 1018 of process 1000 and in blocks 2012, 2018, 2024 and 2030 of process 2000, shown in Figures 13 and 14, respectively. The produced stimulation signals may be square pulses or arbitrary waveforms, constant voltage or constant current. Stimulation location, amplitude, and/or waveform may be adjusted in a closed-loop based on current respiratory conditions or conditions relayed by the respiratory activity monitoring module 1104 in response to previous stimulation. Stimulation waveforms may also contain features allowing for selective stimulation using current steering, directionally selective stimulation of efferent or afferent fibers, selectivity for stimulating axons of a particular diameter, or features designed to block transmission of undesired bioelectric activity.

The stimulation module 1106 may optionally allow remote selection and/or modification of the stimulation strategies and stimulation parameters through a communication link such as, for example, a radio frequency (RF) or infrared (IR) link (not shown).

The stimulation module 1106 may include, without limiting the illustrative embodiment to this component, a pulse generator for providing current and/or voltage stimulation signals to muscles, nerves or tissue. Examples of pulse generators that may be used are disclosed in U.S. Patent Application No. 11/920,814 entitled "IMPLANTABLE PULSE GENERATOR", filed on October 9, 2007, by Roy et al.

Finally, the neuromodulator 1100 may include an internal power supply (not shown) or use a transcutaneous energy transfer system (not shown).

Other applications of the invention will be apparent to those skilled in the art. For example, the device has the capacity to detect respiration rate, phase, and timing. This provides for general monitoring of vital signs, aside from apnea detection, and could provide respiration-related parameters to other devices such as external monitoring equipment, or implanted devices such as pacemakers or implantable defibrillators.

Further, apneas occurring during sleep or waking, as in cases of Cheyne-Stokes respiration or Charcot-Marie-Tooth disease could be effectively treated with the invention described herein. Other adverse respiratory conditions or types of sleep disordered breathing could be detected by monitoring naturally occurring receptors in the airway, such as narrowing or obstruction of the airway, snoring, presence of solids or fluids in the airway, respiratory difficulty in congestive heart failure, presence of reflux in the airway, or inappropriate magnitude or timing of airway muscle activity. Detection of these events might be applied to the detection and treatment of respiratory disorders such as asthma, dysphagia, aspiration pneumonia, or SIDS. Stimulation treatments could result in bronchodilation or bronchoconstriction, change in breathing pattern, swallow, cough, gag, muscle or sphincter activation or inhibition, change in mucus or other secretion, or other activity of the airway.

It is to be understood that the various units, modules and sub-modules and algorithms may be implemented using, for example one or more electronic circuit, microcontroller or DSP.

It is also to be understood that the detection, identification and treatment of sleep apnea processes 1000 (see Figure 13) and 2000 (see Figure 14) as well as the neuromodulator 1100 (see Figure 15) may be selectively activated, for example when a subject is sleeping. The activation may be user initiated, optionally with a delay, according to a given schedule, by monitoring the heart rate of the subject, the orientation of the subject, etc.

Although the present invention has been described by way of illustrative embodiments and examples thereof, it should be noted that it will be apparent to persons skilled in the art that modifications may be applied to the present particular embodiment without departing from the scope of the present invention as defined by the appended claims.

### REFERENCE

[1] Chamberlin, N.L., Eikermann, M., Fassbender, P., White, D.P., and Malhotra, A., (2007) "Genioglossus premotoneurons and the negative pressure reflex in rats." J. Physiol. 579: 515-526.

## Claims

1. A system for monitoring the respiratory activity of a human subject, comprising:
an electrode (1132) configured to be positioned in contact with the internal branch of the superior laryngeal nerve of the subject;
a control unit (1100) operatively connected to the electrode, the implantable control unit including:
a signal conditioning module (1102) for conditioning an electroneurogram signal recorded by the electrode when positioned in contact with the internal branch of the superior laryngeal nerve;
a monitoring and detection module (1104) configured for computing an index of respiratory activity from said electroneurogram signal, said index being indicative of the amplitude of airway pressure of the larynx, and for reporting an occurrence of an apneic event when the index of respiratory activity meets at least one apnea criteria.

2. A system according to claim 1, wherein the index of respiratory activity is indicative of the amplitude and timing of the electroneurogram signal and is selected from a group consisting of the peaks of the electroneurogram signal, the root-mean-square of the electroneurogram signal, the peak-to-peak amplitude of the electroneurogram signal, the duration of peaks of the electroneurogram signal, the interpeak intervals of the electroneurogram signal and any combination thereof.

3. A system according to claim 1, wherein the control unit further includes a stimulation module (1106) operatively connected to the monitoring and detection module, the stimulation module is adapted to generate through the electrode a stimulation signal which acts to increase airway patency or stimulate breathing following the reporting of the apneic event.

4. A system according to claim 3, wherein the at least one apnea criteria includes a first criteria associated with obstructive sleep apnea and a second criteria associated with central sleep apnea, and wherein the monitoring and detection module is adapted to report the apneic event as an obstructive sleep apnea event when the index of respiratory activity meets the first criteria and as a central sleep apnea event when the index of respiratory activity meets the second criteria.

5. A system according to claim 4, wherein the stimulation module is adapted to generate through the electrode a stimulation signal which acts to increase airway patency following the reporting of the obstructive sleep apnea event.

6. A system according to claim 4, wherein the monitoring and detection module is adapted to report an occurrence of an obstructive sleep hypopnea event when the index of respiratory activity meets a third criteria associated with obstructive sleep hypopnea and an occurrence of a central sleep hypopnea event when the index of respiratory activity meets a fourth criteria associated with central sleep hypopnea.

7. A system according to claim 6, wherein the stimulation module is adapted to generate through the electrode a stimulation signal which acts to increase airway patency following the reporting of the obstructive sleep hypopnea event or of the obstructive sleep apnea event, parameters of the stimulation signal being selected according to whether the obstructive sleep hypopnea event or the obstructive sleep apnea event was reported.

8. A system according to claim 3, further comprising a stimulation electrode (1134) operatively connected to the stimulation module, and wherein the stimulation signal is generated through the stimulation electrode.

9. A system according to claim 8, wherein the at least one apnea criteria includes a first criteria associated with obstructive sleep apnea and a second criteria associated with central sleep apnea, and wherein the monitoring and detection module is adapted to report the apneic event as an obstructive sleep apnea event when the index of respiratory activity meets the first criteria and as a central sleep apnea event when the index of respiratory activity meets the second criteria.

10. A system according to claim 9, wherein the stimulation module is adapted to generate through the stimulation electrode a stimulation signal which acts to increase airway patency following the reporting of the obstructive sleep apnea event or stimulate breathing following the reporting of the central sleep apnea event.

11. A system according to claim 8, wherein the monitoring and detection module is adapted to report an occurrence of an obstructive sleep hypopnea event when the index of respiratory activity meets a third criteria associated with obstructive sleep hypopnea and an occurrence of a central sleep hypopnea event when the index of respiratory activity meets a fourth criteria associated with central sleep hypopnea.

12. A system according to claim 11, wherein the stimulation module is adapted to generate through the stimulation electrode a stimulation signal which acts to increase airway patency following the reporting of the obstructive sleep hypopnea event or of the obstructive sleep apnea event, or stimulate breathing following the reporting of the central sleep hypopnea event or of the central sleep apnea event, parameters of the stimulation signal being selected according to whether the obstructive sleep hypopnea event, the obstructive sleep apnea event, the central sleep hypopnea event or the central sleep apnea event was reported.

13. A system according to claim 8, wherein the stimulation electrode is configured to be positioned in contact with the glossopharyngeal nerve of the subject.

14. A system according to any of claims 3, 8 or 13, wherein the stimulation signal is generated by the stimulation module so as to elicit a reflexive pattern activity from the central nervous system of the subject.

15. A system according to claim 14, wherein the reflexive pattern activity is selected from a group consisting of the negative pressure reflex, coughing, yawning, gagging, swallowing and any combination thereof.

16. A system according to claim 15, wherein the swallowing reflexive pattern activity is elicited by generating an electrical signal at a rate of about 20 Hz to 50 Hz.

## Patentansprüche

1. System zum Überwachen der respiratorischen Aktivität eines menschlichen Subjekts, das Folgendes umfasst:
eine Elektrode (1132), die dazu konfiguriert ist, in Kontakt mit dem internen Zweig des oberen Kehlkopfnervs des Subjekts angeordnet zu werden;
eine Steuereinheit (1100), die operativ mit der Elektrode verbunden ist, wobei die implantierbare Steuereinheit Folgendes umfasst:
ein Signalkonditionierungsmodul (1102) zum Konditionieren eines Elektroneurogrammsignals, welches durch die Elektrode aufgezeichnet wird, wenn diese in Kontakt mit dem internen Zweig des oberen Kehlkopfnervs angeordnet ist;
ein Überwachungs- und Detektionsmodul (1104), das dazu konfiguriert ist, einen Index von respiratorischer Aktivität aus dem Elektroneurogrammsignal zu berechnen, wobei der Index kennzeichnend für die Amplitude des Atemwegdrucks des Kehlkopfs ist, und zum Berichten des Auftretens eines Apnoe-Ereignisses, wenn der Index der respiratorischen Aktivität mindestens ein Apnoe-Kriterium erfüllt.

2. System nach Anspruch 1, wobei der Index der respiratorischen Aktivität kennzeichnend für die Amplitude und das Zeitverhalten des Elektroneurogrammsignals ist, und ausgewählt ist aus einer Gruppe bestehend aus den Spitzen des Elektroneurogrammsignals, dem quadratischen Mittel des Elektroneurogrammsignals, der Spitze-zu-Spitze-Amplitude des Elektroneurogrammsignals, der Dauer der Spitzen des Elektroneurogrammsignals, den Zwischenspitzen-Intervallen des Elektroneurogrammsignals, oder einer Kombination derselben.

3. System nach Anspruch 1, wobei die Steuereinheit ferner ein Stimulationsmodul (1106) umfasst, welches operativ mit dem Überwachungs- und Detektionsmodul gekoppelt ist, wobei das Stimulationsmodul dazu geeignet ist, durch die Elektrode ein Stimulationssignal zu erzeugen, welches bewirkt, dass die Atemwegsdurchlässigkeit erhöht oder das Atmen stimuliert wird, folgend dem Bericht des Apnoe-Ereignisses.

4. System nach Anspruch 3, wobei das mindestens eine Apnoe-Kriterium ein erstes Kriterium umfasst, welches mit einer obstruktiven Schlaf-Apnoe assoziiert ist, und ein zweites Kriterium, welches mit einer zentralen Schlafapnoe assoziiert ist, und wobei das Überwachungs- und Detektionsmodul dazu geeignet ist, das Apnoe-Ereignis als ein obstruktives Schlaf-Apnoe-Ereignis zu berichten, wenn der Index der respiratorischen Aktivität das erste Kriterium erfüllt, und als ein zentrales Schlaf-Apnoe-Ereignis, wenn der Index der respiratorischen Aktivität das zweite Kriterium erfüllt.

5. System nach Anspruch 4, wobei das Stimulationsmodul dazu geeignet ist, durch die Elektrode ein Stimulationssignal zu erzeugen, welches bewirkt, dass die Atemwegsdurchlässigkeit erhöht wird, folgend dem Bericht des obstruktiven Schlaf-Apnoe-Ereignisses.

6. System nach Anspruch 4, wobei das Überwachungs- und Detektionsmodul dazu geeignet ist, das Auftreten eines obstruktiven Schlaf-Hypopnoe-Ereignisses zu berichten, wenn der Index der respiratorischen Aktivität ein drittes Kriterium erfüllt, welches mit einer obstruktiven Schlaf-Hypopnoe assoziiert ist, und das Auftreten eines zentralen Schlaf-Hypopnoe-Ereignisses, wenn der Index der respiratorischen Aktivität ein viertes Kriterium erfüllt, welches mit einer zentralen Schlaf-Hypopnoe assoziiert ist.

7. System nach Anspruch 6, wobei das Stimulationsmodul dazu geeignet ist, durch die Elektrode ein Stimulationssignal zu erzeugen, welches bewirkt, dass die Atemwegsdurchlässigkeit erhöht wird, folgend dem Bericht des obstruktiven Schlaf-Hypopnoe-Ereignisses oder des obstruktiven Schlaf-Apnoe-Ereignisses, wobei Parameter des Stimulationssignals abhängig davon ausgewählt werden, ob das obstruktive Schlaf-Hypopnoe-Ereignis oder das obstruktive Schlaf-Apnoe-Ereignis berichtet wurde.

8. System nach Anspruch 3, das ferner eine Stimulationselektrode (1134) umfasst, die operativ mit dem Stimulationsmodul gekoppelt ist, und wobei das Stimulationssignal durch die Stimulationselektrode erzeugt wird.

9. System nach Anspruch 8, wobei das mindestens eine Apnoe-Kriterium ein erstes Kriterium umfasst, welches mit einer obstruktiven Schlaf-Apnoe assoziiert ist, und ein zweites Kriterium, welches mit einer zentralen Schlaf-Apnoe assoziiert ist, und wobei das Überwachungs- und Detektionsmodul dazu geeignet ist, das Apnoe-Ereignis als ein obstruktives Schlaf-Apnoe-Ereignis zu berichten, wenn der Index der respiratorischen Aktivität das erste Kriterium erfüllt, und als ein zentrales Schlaf-Apnoe-Ereignis, wenn der Index der respiratorischen Aktivität das zweite Kriterium erfüllt.

10. System nach Anspruch 9, wobei das Stimulationsmodul dazu geeignet ist, durch die Stimulationselektrode ein Stimulationssignal zu erzeugen, welches bewirkt, dass die Atemwegsdurchlässigkeit erhöht wird, folgend dem Bericht des obstruktiven Schlaf-Apnoe-Ereignisses, oder das Atmen stimuliert, folgend dem Bericht des zentralen Schlaf-Apnoe-Ereignisses.

11. System nach Anspruch 8, wobei das Überwachungs- und Detektionsmodul dazu geeignet ist, das Auftreten eines obstruktiven Schlaf-Hypopnoe-Ereignisses zu berichten, wenn der Index der respiratorischen Aktivität ein drittes Kriterium erfüllt, welches mit einer obstruktiven Schlaf-Hypopnoe assoziiert ist, und das Auftreten eines zentralen Schlaf-Hypopnoe-Ereignisses, wenn der Index der respiratorischen Aktivität ein viertes Kriterium erfüllt, welches mit der zentralen Schlaf-Hypopnoe assoziiert ist.

12. System nach Anspruch 11, wobei das Stimulationsmodul dazu geeignet ist, durch die Stimulationselektrode ein Stimulationssignal zu erzeugen, welches bewirkt, dass die Atemwegsdurchlässigkeit erhöht wird, folgend dem Bericht des obstruktiven Schlaf-Hypopnoe-Ereignisses oder des obstruktiven Schlaf-Apnoe-Ereignisses, oder die Atmung nachfolgend dem Bericht des zentralen Schlaf-Hypopnoe-Ereignisses oder des zentralen Schlaf-Apnoe-Ereignisses zu stimulieren, wobei Parameter des Stimulationssignals in Abhängigkeit davon ausgewählt werden, ob das obstruktive Schlaf-Hypopnoe-Ereignis, das obstruktive Schlaf-Apnoe-Ereignis, das zentrale Schlaf-Hypopnoe-Ereignis oder das zentrale Schlaf-Apnoe-Ereignis berichtet wurde.

13. System nach Anspruch 8, wobei die Stimulationselektrode dazu konfiguriert ist, in Kontakt mit dem Zungen-Rachen-Nerv positioniert zu werden.

14. System nach einem der Ansprüche 3, 8 oder 13, wobei das Stimulationssignal durch das Stimulationsmodul so erzeugt wird, dass es eine reflexive Musteraktivität von dem zentralen Nervensystem des Subjekts anregt.

15. System nach Anspruch 14, wobei die reflexive Musteraktivität ausgewählt ist aus einer Gruppe bestehend aus einem Unterdruckreflex, Husten, Gähnen, Würgen, Schlucken oder einer Kombination derselben.

16. System nach Anspruch 15, wobei die dem Schlucken entsprechende reflexive Musteraktivität dadurch angeregt wird, dass ein elektrisches Signal mit einer Rate von ungefähr 20 Hz bis 50 Hz erzeugt wird.

## Revendications

1. Système pour surveiller l'activité respiratoire d'un sujet humain, comprenant :
une électrode (1132) configurée pour être positionnée en contact avec la branche interne du nerf laryngé supérieur du sujet ;
une unité de commande (1100) connectée opérationnellement à l'électrode, l'unité de commande implantable incluant :
un module de conditionnement de signal (1102) pour conditionner un signal d'électroneurogramme enregistré par l'électrode lorsqu'elle est positionnée en contact avec la branche interne du nerf laryngé supérieur ;
un module de surveillance et de détection (1104) configuré pour calculer un indice d'activité respiratoire à partir dudit signal d'électroneurogramme, ledit indice étant indicateur de l'amplitude de la pression des voies aériennes du larynx, et pour rapporter une occurrence d'un événement apnéique lorsque l'indice d'activité respiratoire répond à au moins un critère d'apnée.

2. Système selon la revendication 1, dans lequel l'indice d'activité respiratoire est indicateur de l'amplitude et la synchronisation du signal d'électroneurogramme et est sélectionné dans un groupe constitué des crêtes du signal d'électroneurogramme, de la valeur quadratique moyenne du signal d'électroneurogramme, de l'amplitude crête à crête du signal d'électroneurogramme, de la durée des crêtes du signal d'électroneurogramme, des intervalles entre crêtes du signal d'électroneurogramme et de toute combinaison de ceux-ci.

3. Système selon la revendication 1, dans lequel l'unité de commande inclut en outre un module de stimulation (1106) connecté de manière opérationnelle au module de surveillance et de détection, le module de stimulation est adapté pour générer à travers l'électrode un signal de stimulation qui agit pour augmenter la perméabilité des voies aériennes ou stimuler la respiration suite au rapport de l'événement apnéique.

4. Système selon la revendication 3, dans lequel l'au moins un critère d'apnée inclut un premier critère associé à l'apnée obstructive du sommeil et un deuxième critère associé à l'apnée centrale du sommeil, et dans lequel le module de surveillance et de détection est adapté pour rapporter l'événement apnéique comme un événement d'apnée obstructive du sommeil lorsque l'indice d'activité respiratoire répond au premier critère et comme un événement d'apnée centrale du sommeil lorsque l'indice d'activité respiratoire répond au deuxième critère.

5. Système selon la revendication 4, dans lequel le module de stimulation est adapté pour générer à travers l'électrode un signal de stimulation qui agit pour augmenter la perméabilité des voies aériennes suite au rapport de l'événement d'apnée obstructive du sommeil.

6. Système selon la revendication 4, dans lequel le module de surveillance et de détection est adapté pour rapporter une occurrence d'un événement d'hypopnée obstructive du sommeil lorsque l'indice d'activité respiratoire répond à un troisième critère associé à l'hypopnée obstructive du sommeil et une occurrence d'un événement d'hypopnée centrale du sommeil lorsque l'indice d'activité respiratoire répond à un quatrième critère associé à l'hypopnée centrale du sommeil.

7. Système selon la revendication 6, dans lequel le module de stimulation est adapté pour générer à travers l'électrode un signal de stimulation qui agit pour augmenter la perméabilité des voies aériennes suite au rapport de l'événement d'hypopnée obstructive du sommeil ou de l'événement d'apnée obstructive du sommeil, les paramètres du signal de stimulation étant sélectionnés selon que l'événement d'hypopnée obstructive du sommeil ou l'événement d'apnée obstructive du sommeil a été rapporté.

8. Système selon la revendication 3, comprenant en outre une électrode de stimulation (1134) connectée de manière opérationnelle au module de stimulation, et dans lequel le signal de stimulation est généré à travers l'électrode de stimulation.

9. Système selon la revendication 8, dans lequel l'au moins un critère d'apnée inclut un premier critère associé à l'apnée obstructive du sommeil et un deuxième critère associé à l'apnée centrale du sommeil, et dans lequel le module de surveillance et de détection est adapté pour rapporter l'événement apnéique comme un événement d'apnée obstructive du sommeil lorsque l'indice d'activité respiratoire répond au premier critère et comme un événement d'apnée centrale du sommeil lorsque l'indice d'activité respiratoire répond au deuxième critère.

10. Système selon la revendication 9, dans lequel le module de stimulation est adapté pour générer à travers l'électrode de stimulation un signal de stimulation qui agit pour augmenter la perméabilité des voies aériennes suite au rapport de l'événement d'apnée obstructive du sommeil ou pour stimuler la respiration suite au rapport de l'événement d'apnée centrale du sommeil.

11. Système selon la revendication 8, dans lequel le module de surveillance et de détection est adapté pour rapporter une occurrence d'un événement d'hypopnée obstructive du sommeil lorsque l'indice d'activité respiratoire répond à un troisième critère associé à l'hypopnée obstructive du sommeil et une occurrence d'un événement d'hypopnée centrale du sommeil lorsque l'indice d'activité respiratoire répond à un quatrième critère associé à l'hypopnée centrale du sommeil.

12. Système selon la revendication 11, dans lequel le module de stimulation est adapté pour générer à travers l'électrode de stimulation un signal de stimulation qui agit pour augmenter la perméabilité des voies aériennes suite au rapport de l'événement d'hypopnée obstructive du sommeil ou de l'événement d'apnée obstructive du sommeil, ou pour stimuler la respiration suite au rapport de l'événement d'hypopnée centrale du sommeil ou de l'événement d'apnée centrale du sommeil, les paramètres du signal de stimulation étant sélectionnés selon que l'événement d'hypopnée obstructive du sommeil, l'événement d'apnée obstructive du sommeil, l'événement d'hypopnée centrale du sommeil ou l'événement d'apnée centrale du sommeil a été rapporté.

13. Système selon la revendication 8, dans lequel l'électrode de stimulation est configurée pour être positionnée en contact avec le nerf glossopharyngé du sujet.

14. Système selon l'une quelconque des revendications 3, 8 ou 13, dans lequel le signal de stimulation est généré par le module de stimulation de sorte à susciter une activité de modèle réflexif à partir du système nerveux central du sujet.

15. Système selon la revendication 14, dans lequel l'activité de modèle réflexif est sélectionnée à partir du groupe constitué du réflexe de pression négative, de la toux, du bâillement, de la déglutition et de toute combinaison de ceux-ci.

16. Système selon la revendication 15, dans lequel l'activité de modèle réflectif de la déglutition est suscitée en générant un signal électrique à une fréquence d'environ 20 Hz à 50 Hz.
